# EUROPÄISCHE PATENTSCHRIFT

(11) **EP 0 763 036 B1**
(45) Veröffentlichungstag und Bekanntmachung des Hinweises auf die Patenterteilung: **11.09.2002**
(21) Anmeldenummer: 95921799.3
(22) Anmeldetag: 31.05.1995
(51) Int. Cl.: C07D 487/04, A61K 31/40, A61K 31/425, C07D 517/04, C07D 471/04, C07D 498/04, C07F 9/6561

(54) **[a]-ANNELIERTE PYRROLDERIVATE UND DEREN ANWENDUNG IN DER PHARMAZIE**
[a]-ANNELATED PYRROLE DERIVATIVES AND PHARMACEUTICAL USE THEREOF
DERIVES DE PYRROLE [a]-ANNELES ET LEUR UTILISATION DANS LE DOMAINE PHARMACEUTIQUE

(30) Priorität: 01.06.1994 DE 4419246
(43) Veröffentlichungstag der Anmeldung: 19.03.1997
(73) Patentinhaber: MERCKLE GMBH, 89143 Blaubeuren (DE)
(72) Erfinder: LAUFER, Stefan, D-89143 Blaubeuren (DE); STRIEGEL, Hans, Günther, D-89143 Blaubeuren (DE); DANNHARDT, Gerd, D-55127 Mainz (DE)
(74) Vertreter: Kinzebach, Werner, Dr.
(86) Internationale Anmeldenummer: EP9502077
(87) Internationale Veröffentlichungsnummer: WO95032970

(56) Entgegenhaltungen:
- EP-A- 0 118 321
- EP-A- 0 147 317
- EP-A- 0 397 175

## Beschreibung

Die Erfindung betrifft Pyrrole, die an Bindung a anneliert sind, und deren Anwendung in der Pharmazie sowie pharmazeutische Mittel, welche diese Verbindungen enthalten.

Es ist bekannt, daß die Metabolisierung von Arachidonsäure auf zwei verschiedenen Wegen erfolgt. Beim Cyclooxygenase-Weg wird unter Einwirkung des Enzyms Cyclooxygenase die Arachidonsäure zu Prostaglandinen metabolisiert. Beim Lipoxygenase-Weg wird die Arachidonsäure unter Einwirkung von Lipoxygenasen zu den sogenannten Leukotrienen metabolisiert.

Die Prostaglandine sind an der Entstehung von Entzündung, Fieber und Schmerz beteiligt, während die Leukotriene bei der Entstehung von Asthma, Entzündungen und Allergien eine wichtige Rolle spielen. Zur Bekämpfung dieser Symptome werden häufig nicht-steroidale Antiphlogistika, wie Arylessigsäure-, 2-Arylpropionsäure- und Anthranilsäure-Derivate, eingesetzt. Diese Derivate hemmen die Cyclooxygenase und verhindern somit die Bildung der Prostaglandine aus Arachidonsäure. Die Anwendung derartiger Derivate ist jedoch aufgrund ihrer Nebenwirkungen nicht unbedenklich. Arzneimittel, welche die Lipoxygenase hemmen, sind im Handel jedoch nicht erhältlich.

Die EP-A-397 175 beschreibt Pyrrolizinverbindungen der Formel: 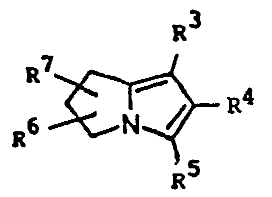 worin zwei der Reste R³, R⁴ und R⁵ unabhängig voneinander für H, C₅-C₈-Cycloalkyl, C₁-C₁₂-Alkyl oder Aryl, das gegebenenfalls durch ein oder zwei Reste substituiert ist, die ausgewählt sind unter Halogen, NO₂, C₁-C₄-Alkoxy, Hydroxy, C₁-C₄-Alkyl oder Phenoxy, stehen und der dritte der Reste R³, R⁴ und R⁵ für CHO, CO₂H, COSC₁-C₄-Alkyl oder A - X steht, wobei A eine geradkettige oder verzweigte C₁-C₈-Alkylengruppe oder eine C₂-C₈-Alkenylengruppe bedeutet und X für CO₂H, SO₃H, CHO, OH oder SH steht. Diese Verbindungen sind Cyclooxygenase- und/oder Lipoxygenasehemmer und daher bei der Behandlung von Erkrankungen des rheumatischen Formenkreises und zur Prävention von allergisch induzierten Erkrankungen brauchbar.

Die EP 147 317 A beschreibt Verbindungen der Formel 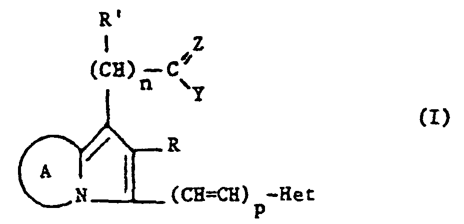 worin Het für 3-Pyridyl oder 5-Thiazolyl steht, p für 0 oder 1 steht, Z u.a. für O steht, Y für einen Aminorest oder OH steht, n für 0 oder 1 steht, R' u.a. für ein Wasserstoffatom steht und der Ring A zusammen mit dem Pyrrolring u.a. einen Pyrrolizin- oder Pyrrolothiazolring bilden kann. Ähnliche Verbindungen sind in der EP 118 321 A beschrieben. Die in diesen Anmeldungen beschriebenen Verbindungen sind zur prophylaktischen und therapeutischen Behandlung von thrombotischen Erkrankungen brauchbar.

Überraschenderweise wurde nun gefunden, daß bestimmte heterocyclische Verbindungen den oben beschriebenen Pyrrolizinverbindungen in ihrer Wirkung und insbesondere hinsichtlich der analgetischen Wirkkomponente überlegen sind und außerdem cholesterolsenkende Wirkung besitzen.

Gegenstand der Erfindung sind daher heterocyclische Verbindungen der Formel I: 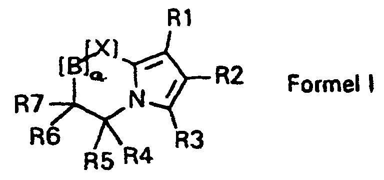 worin
einer der Reste R¹, R² und R³ für einen Thiophen-, Furan-, Benzofuran- oder Pyrimidinrest steht, der gegebenenfalls durch Halogen oder C₁-C₈-Alkyl substituiert ist,
der zweite der Reste R¹, R² und R³ für ein Wasserstoffatom oder einen Phenyl- oder Naphthylrest steht, der gegebenenfalls einen oder zwei Substituenten aufweist, die unter Halogen, Pseudohalogen, CF₃ und C₁-C₈-Alkyl ausgewählt sind, und
der dritte der Reste R¹, R² und R³ für H, CHO, CO₂H, COSC₁-C₈-Alkyl, COCO₂H, oder A - Y steht,
A für C₁-C₈-Alkylen oder C₂-C₈-Alkenylen steht,
Y für CO₂H, SO₃H, OPO(OH)₂, OP(OH)₂, eine Tetrazolylgruppe, CHO oder OH steht,
R⁴, R⁵, R⁶ und R⁷, die gleich oder verschieden sein können, für H oder C₁-C₈-Alkyl stehen oder zwei dieser Reste für eine chemische Bindung zwischen den beiden Ringatomen, an die sie gebunden sind, stehen und die beiden anderen die angegebenen Bedeutungen besitzen,
X für CH₂ oder S steht,
B für CH₂ steht, und
a für 0, 1 oder 2 steht, und
die optischen Isomere, Salze und C₁-C₈-Alkyl-, Pivaloyloxymethyl-, Acetoxymethyl-, Phthalidyl-, Indanyl- oder Methoxymethylester davon.

Die pharmazeutisch verträglichen Salze können im vorliegenden Fall Säureadditions- oder Basenadditionssalze sein. Für Säureadditionssalze verwendet man anorganische Säuren, wie Salzsäure, Schwefelsäure oder Phosphorsäure, oder organische Säuren, wie Weinsäure, Milchsäure, Citronensäure, Äpfelsäure, Mandelsäure, Ascorbinsäure, Maleinsäure, Fumarsäure, Gluconsäure und dergleichen.

Zu Basenadditionssalzen zählen Salze der Verbindungen der Formel I mit anorganischen Basen, wie Natrium- oder Kaliumhydroxid oder mit organischen Basen, wie Mono-, Di- oder Triethanolamin.

Zu den Estern der Verbindungen der Formel I zählen insbesondere physiologisch leicht hydrolysierbare Ester, beispielsweise Alkyl-, Pivaloyloxymethyl-, Acetoxymethyl-, Phthalidyl-, Indanyl- und Methoxymethylester.

Der Ausdruck "Alkyl, Alkoxy etc." umfaßt geradkettige oder verzweigte Alkylgruppen, wie Methyl, Ethyl, n- und i-Propyl, n-, i- oder t-Butyl, n-Pentyl, Neopentyl, n-Hexyl etc.

Soweit nicht anders angegeben, steht "Alkyl" vorzugsweise für C₁-C₈-Alkyl, insbesondere für C₁-C₆-Alkyl und besonders bevorzugt für C₁-C₄-Alkyl.

"Aryl" steht vorzugsweise für Naphthyl und insbesondere für Phenyl.

Der Ausdruck "Halogenatom" umfaßt ein Fluor-, Chlor-, Bromoder Jodatom und insbesondere ein Fluor- oder Chloratom. "Pseudohalogen" steht insbesondere für CN, OCN, SCN oder N₃.

"Alkylen" oder "Alkenylen" steht für geradkettige oder verzweigte Alkylen- oder Alkenylengruppen mit vorzugsweise 1 bis 6 bzw. 2 bis 6 und insbesondere 1 bis 4 bzw. 2 bis 4 Kohlenstoffatomen. Die Alkylengruppe und insbesondere die Methylengruppe ist bevorzugt.

Eine ein Säureequivalent darstellende Gruppe ist insbesondere die Tetrazolylgruppe.
"Acyl" steht für RCO, wobei R vorzugsweise die oben für "Alkyl" und "Aryl" angegebenen Bedeutungen besitzt. Acetyl ist besonders bevorzugt.

Bei dem "aromatischen heterocyclischen Rest" handelt es sich insbesondere um einen Thiophen-, Furan-, Pyrimidin-oder Benzofuranrest. Wenn der Heterozyklus substituiert ist, sind 1, 2 oder 3 Substituenten vorhanden, die ausgewählt sind unter Halogen oder C₁-C₈-Alkyl. Bevorzugt ist ein Thiophen- oder halogen-, insbesondere chlorsubstituierter Thiophenrest, ein Furan- oder Benzofuranrest.

Wenn einer der Reste R¹, R² und R³ einen heterocyclischen Rest oder einen substituierten Arylrest bedeutet, dann steht vorzugsweise R² für einen derartigen Rest.

Die Substituenten der Arylgruppe sind vorzugsweise ausgewählt unter Halogen, insbesondere Fluor oder Chlor, und CF₃. Wenn es sich bei der Arylgruppe um einen Phenylring handelt, befinden sich die Substituenten vorzugsweise in m- und/oder p-Stellung.

Eine bevorzugte Ausführungsform sind die Verbindungen der Formel I, worin einer der Reste R¹, R² und R³ für den erwähnten heterocyclischen Rest steht und der zweite für Phenyl, durch ein bis drei Halogenatome substituiertes Phenyl, Thienyl oder halogensubstituiertes Thienyl steht.

Vorzugsweise steht der dritte der Reste R¹, R² und R³ in 5-Position des Pyrrolizidingerüstes. Insbesondere steht R³ für A - Y.

Eine weiterhin bevorzugte Ausführungsform sind die Verbindungen der obigen Formel I, worin R¹ für Phenyl steht, R² für einen 5-oder 6-gliedrigen heterocyclischen Ring steht und R³ für A - Y steht, wobei A und Y die oben angegebenen Bedeutungen besitzen.

Vorzugsweise stehen A für C₁-C₈-Alkylen und Y für CO₂H, CO₂C₁-C₈-Alkyl, SO₃H, SO₃C₁-C₈-Alkyl, COCO₂H oder COCO₂C₁-C₈-Alkyl.

Besonders bevorzugt steht A - Y für CH₂COOH. a steht vorzugsweise für O.

Eine Ausführungsform sind die Verbindungen der obigen Formel I, worin zwei der Reste R⁴ und R⁶ bzw. R⁵ und R⁷ zusammen für eine chemische Bindung stehen oder worin die Reste R⁴ bis R⁶ für H oder C₁-C₈-Alkyl stehen. Diese Verbindungen besitzen die Formel:

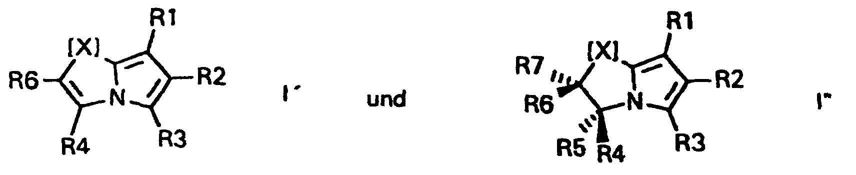

Die Reste R¹ bis R⁷ und X besitzen dabei die oben angegebenen Bedeutungen.

Gemäß einer weiteren bevorzugten Ausführungsform stehen in der Formel I" R⁶ und R⁷ für C₁-C₈-Alkyl und R⁴ und R⁵ für Wasserstoff, wenn X für CH₂ steht; und R⁶ und R⁷ für H und R⁴ und/oder R⁵ für C₁-C₈-Alkyl, wenn X für S steht.

Soweit die erfindungsgemäßen Verbindungen Asymmetriezentren aufweisen, sind Racemate sowie optische Isomere (Enantiomere, Diastereomere) umfaßt.

Die Synthese der erfindungsgemäßen Verbindungen erfolgt analog zu den in den Fig. 1a-c, 2, 3a, 3b, 4, 5a und 5b beschriebenen Verfahren. Diese Verfahren sind zum Teil in der EP-A-397 175 beschrieben; auf diese Publikation einschließlich der darin erwähnten Literaturzitate, wird hiermit Bezug genommen.

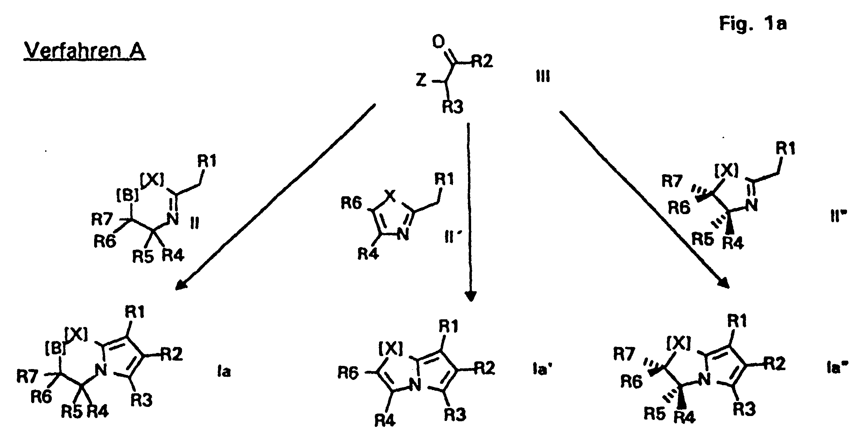

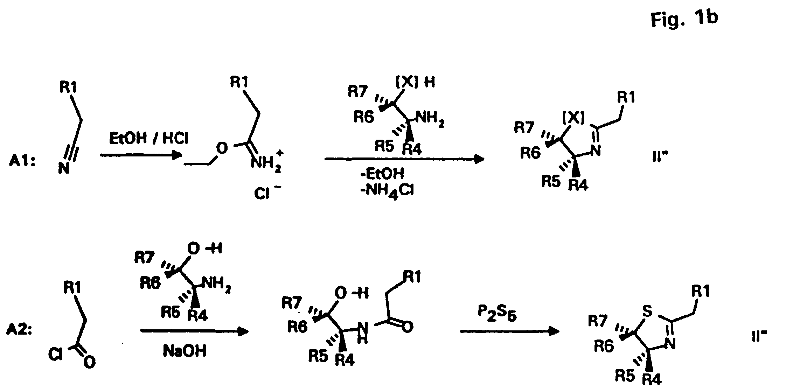

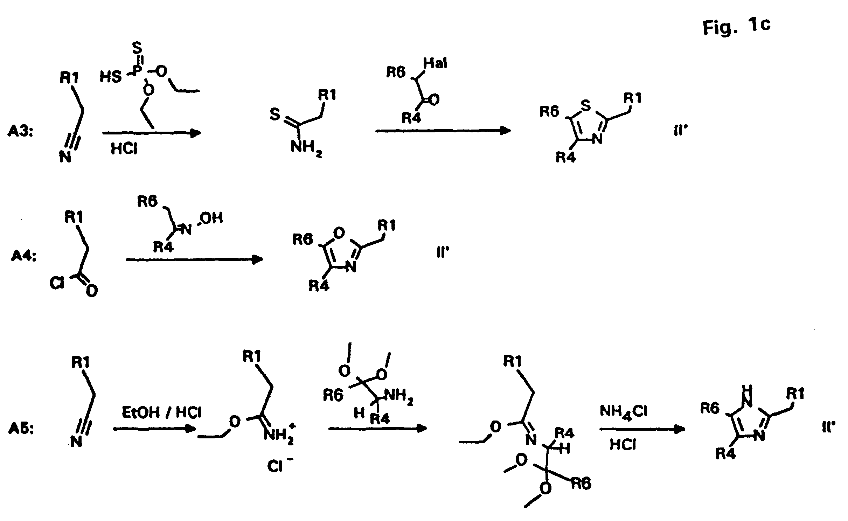

Ausgangsverbindungen für die Herstellung der erfindungsgemäßen Verbindungen sind die Verbindungen der Formel II: 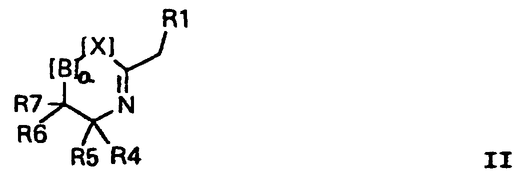 worin R¹, R⁴ bis R⁷ und X die oben angegebenen Bedeutungen besitzen. Diese Verbindungen sind literaturbekannt oder sie werden analog zu bekannten Verfahren hergestellt, z.B. durch die in EP -A-397 175 beschriebenen (X = CH₂, CO) oder durch Umsetzung der von D- und L-Aminosäuren abgeleiteten Aminoalkohole, von Aminothiolen und von Diaminen mit den Imidestern entsprechend substituierter Carbonsäuren (Fig. 1b:A1/A2).

Die Verbindungen der Formel II werden mit den entsprechenden Verbindungen der Formel III 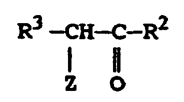 worin Z für Cl oder Br steht und R² und R³ die gewünschten Bedeutungen besitzen, umgesetzt, siehe Verfahren A. Die Verbindungen der Formel III sind ebenfalls literaturbekannt, teils kommerziell erhältlich oder sie werden analog zu bekannten Verfahren aus kommerziell erhältlichen Vorstufen hergestellt, beispielsweise werden entsprechende Acetophenone, Arylacetaldehyde oder Deoxybenzoine mit Brom behandelt oder entsprechende Arylverbindungen und aromatische heterocyclische Verbindungen mit Chloracetylchlorid/AlCl₃ behandelt (vgl.: J.J. Riehl in C.R.Hebd. Seance Acad. Sci Ser. C (1957), 245, 1321-1322). Die Umsetzung erfolgt in einem inerten Lösungsmittel (z.B. Ethanol, Methylenchlorid, Diethylether, Tetrahydrofuran) in Gegenwart einer geeigneten Base (z.B. NaHCO₃, Triethylamin). Wenn X für S steht, erfolgt die Umsetzung vorzugsweise in einem Ether oder aromatischen Kohlenwasserstoff, z.B. Diethylether, Benzol oder Toluol, wobei in der Regel das quaternisierte Zwischenprodukt ausfällt. Dieses wird isoliert und in einem chlorierten Lösungsmittel, z.B. CH₂Cl₂, gelöst und mit einer Base, z.B. Triethylamin, behandelt.

Man erhält aus dieser Umsetzung die Verbindungen der Formel Ia: 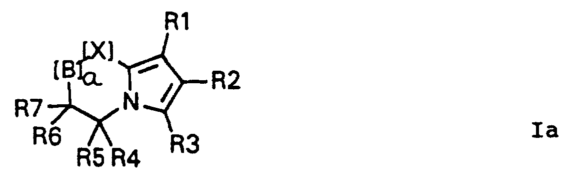 Wenn dabei mindestens einer der Reste R¹, R² und R³ für ein Wasserstoffatom steht, erhält man die Verbindungen der Formeln IVa bis IVc:

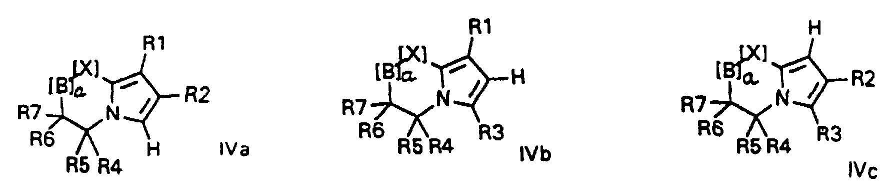

Je nach Stellung des Wasserstoffatoms leiten sich hiervon die Verbindungen der Reihe a, b oder c ab.

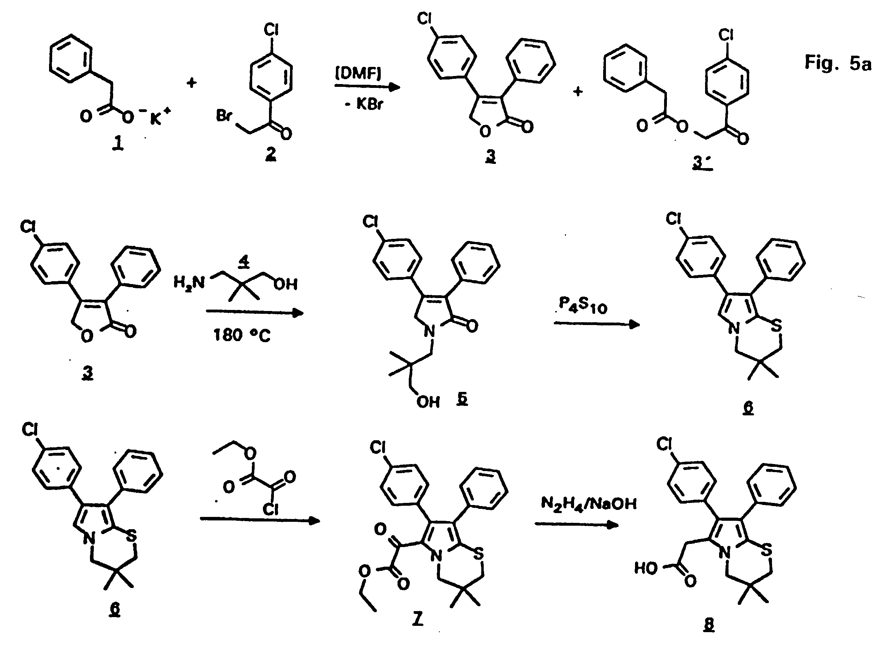

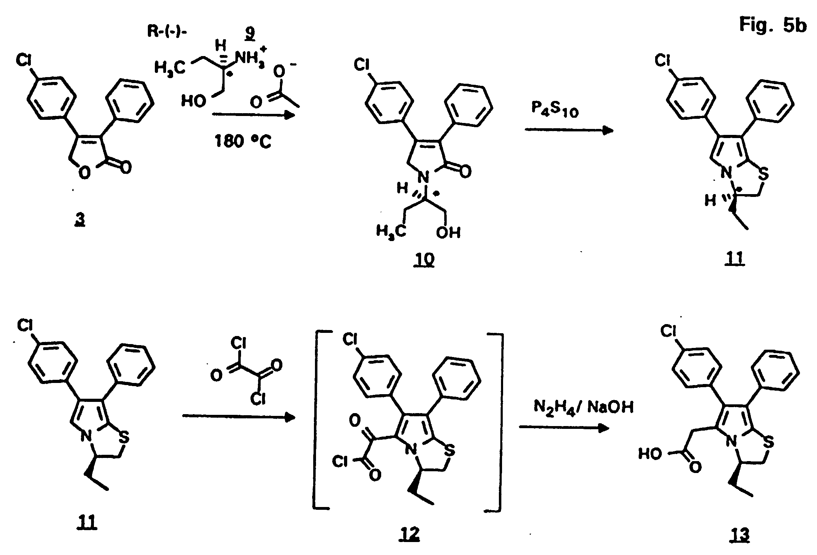

Diese Reaktion sowie die nachfolgend erwähnten Reaktionen sind in den Fig. 1a-c, 2, 3a, 3b, 4,5a und 5b am Beispiel der Verbindungen der Reihe a skizziert. Analoges gilt für die Synthese und Derivatbildung der Verbindungen der Reihen b und c.

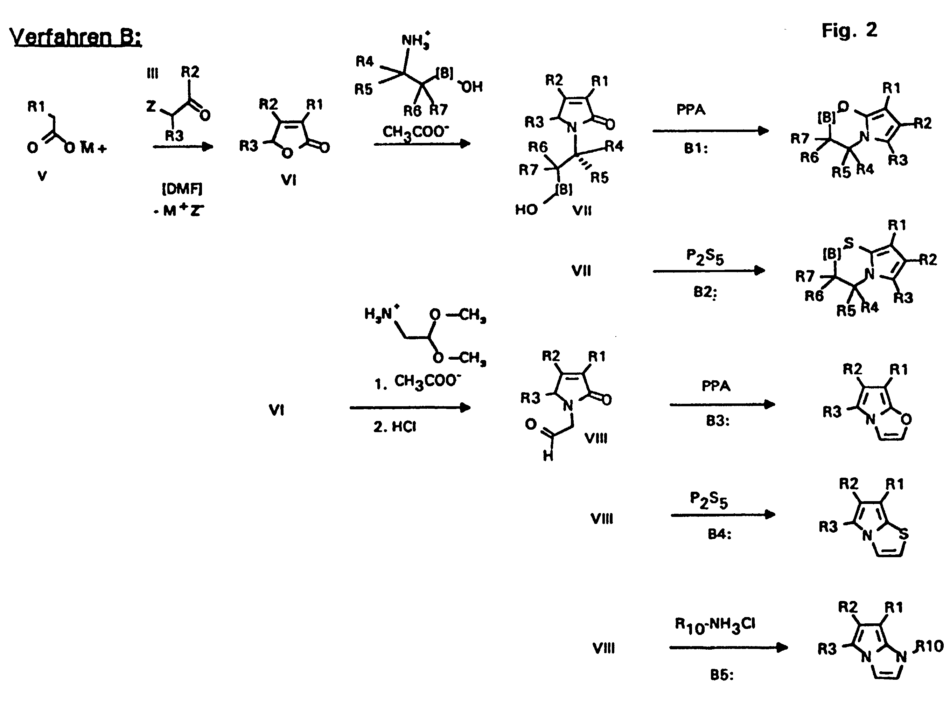

Zusätzlich zu dem in EP A-397 175 beschriebenen Verfahren (Verfahren A) findet zum Aufbau der Heterocyclen IVa, IVb und IVc mit X = S ein weiteres Verfahren (Verfahren B) Anwendung (Fig. 2): Ausgangspunkt dieses Verfahrens sind entsprechend substituierte 2-(5H)Furanone (VI) , die aus Carbonsäuresalzen der Struktur V und den Halogenaldehyden und -ketonen der Struktur III hergestellt werden (Fig. 2), analog zu den in der Literatur beschriebenen Methoden (a: Rio, G. und Sekiz, B. Bull. Soc. Chim. Fr. **1976**, 1491, 1495. b: Padwa, A. , Brookhart, T., Dehm, D. und Wubbels, G., J. Am. Chem. Soc. **1978**, *100,* 8247, 8259). Analog zu literaturbekannten Methoden werden diese in 1,5-Dihydro-2-pyrrolone (VII bzw. VIII) umgewandelt (c: Matsuda et al. Yakugaku Zasshi *95*,[**1975**] 190, 194 (C.A. 83 [1975] 42 780; d: Rio, G. und Sekiz, B., **s.o.**).

Die Cyclisierung zum annelierten Heterocyclus führt - abhängig vom verwendeten Kondensationsreagenz und von der zweiten funktionellen Gruppe des im vorhergehenden Schritt eingeführten bifunktionellen Amins NH₂-CR₄R₅CR₆R₇-[B]ₐ-OH bzw NH₂CH₂CH(OCH₃)₂ - zu teilhydrierten (Formel I", Fig. 2: B1 /B2) bzw. zu dehydrierten Formen (Formel I', Fig. 2: B3, B4, B5).

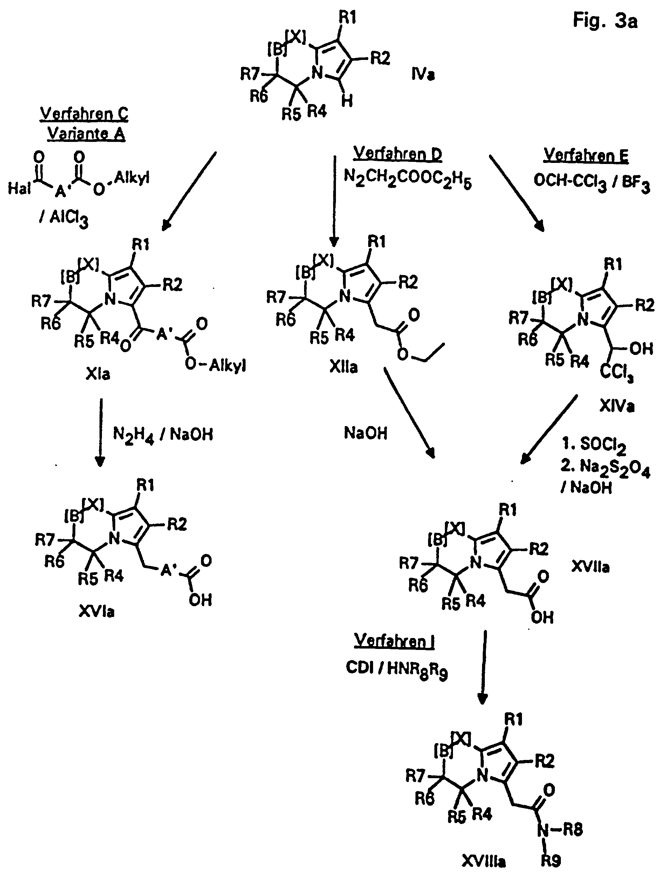

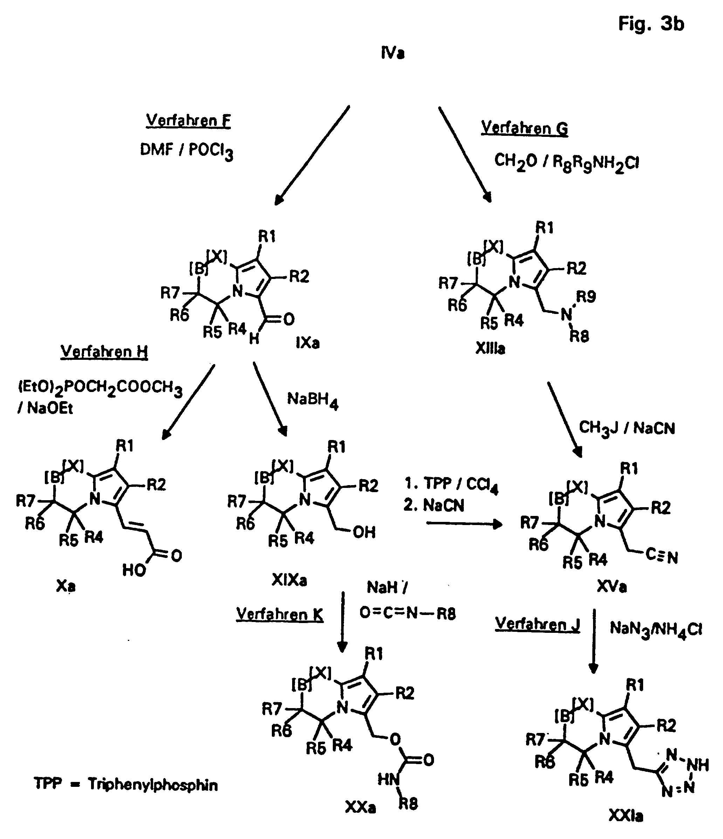

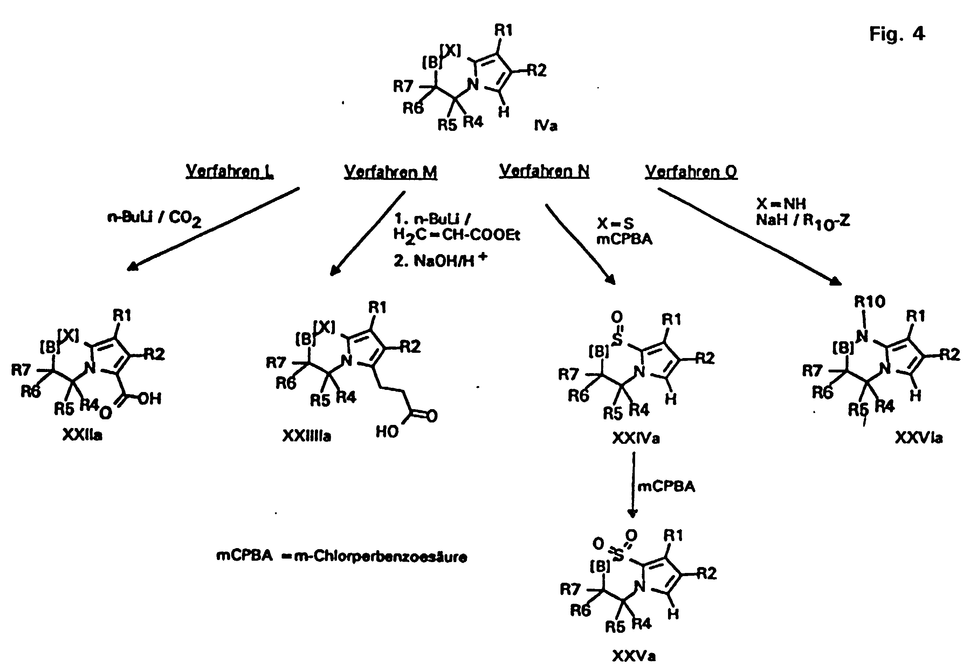

Gewünschtenfalls wird dann in das heterocyclische Grundgerüst ein weiterer Substituent nach Methoden eingeführt, die dem Fachmann bekannt sind. Zu diesen Methoden zählen beispielsweise:
a) Umsetzung einer Verbindung der Formel IV mit einem Carbonsäurehalogenid HalOC-A'-COOAlkyl, worin A' für eine chemische Bindung, C₁-C₇-Alkylen oder C₂-C₇-Alkenylen steht und Hal für Cl oder Br steht. (Fig. 3a, Verfahren C/Variante A). Die erhaltene Verbindung der Formel I, worin einer der Reste R¹, R² und R³ für CO-A'-CO₂Alkyl steht, wird dann mit einem Reagens behandelt, das zur Reduktion der Carbonylgruppe zu einer CH₂-Gruppe geeignet ist, beispielsweise Hydrazin, NaCNBH₃ oder Zink-Amalgam.
   Die Umsetzung mit dem Carbonsäurehalogenid wird in einem inerten Lösungsmittel, z.B. Diethylether oder Tetrahydrofuran, gegebenenfalls in Gegenwart eines Katalysators durchgeführt. Die Reduktion mit Hydrazin erfolgt bevorzugt in einem hochsiedenden Alkohol, z.B. Diethylenglycol. Man erhält auf diese Weise die Verbindungen XVI.
b) Zur Einführung der besonders bevorzugten Gruppe CH₂CO₂H stehen mehrere Methoden zur Verfügung (siehe Fig. 3a, 3b und 4). Die erste Möglichkeit besteht darin, daß man eine Verbindung der Formel IV mit Oxalylchlorid umsetzt (Fig. 5b), wobei man eine Verbindung der Formel XI erhält, in der einer der Reste R¹, R² und R³ für COCO₂H steht. Diese Verbindung wird dann mit einem Reagens behandelt, das zur Reduktion der Ketocarbonylgruppe geeignet ist, beispielsweise Hydrazin, NaCNBH₃ oder Zink-Amalgam. Bevorzugt ist die Reduktion mit Hydrazin unter den Bedingungen einer Wolff-Kishner-Reduktion und insbesondere der Huang-Minlon-Variante davon (vgl. auch Punkt a) oben).
   Eine weitere Möglichkeit besteht darin, eine Verbindung der Formel IV mit einem Diazoessigsäurealkylester zu einer Verbindung der Formel Ic umzusetzen, in der einer der Reste R¹, R² und R³ für CH₂COOAlkyl steht. Diese Verbindung wird dann gewünschtenfalls einer Esterspaltung zu der entsprechenden freien Carbonsäure unterworfen (Fig. 3a, XVII).
   Die Umsetzung mit dem Diazoessigester erfolgt in einem inerten Lösungsmittel, beispielsweise Toluol oder Xylol in Anwesenheit von Kupferpulver oder komplexen Kupfer-Isalzen oder Kupfer-II-salzen. Die Reaktion wird bei erhöhter Temperatur durchgeführt, zweckmäßigerweise bei der Siedetemperatur des verwendeten Lösungsmittels.
   Eine weitere Möglichkeit besteht in der Umsetzung einer Verbindung der Formel IV mit Chloral zu einer Verbindung der Formel XIV und Behandlung der aktivierten Verbindung mit einem Dithionit, beispielsweise Natriumdithionit oder einem Sulfinat, z.B. Hydroxymethansulfinsäure-Natriumsalz, siehe Fig. 3, Verfahren E.
c) Die Einführung einer Formyl- oder Methylolgruppe in den Pyrrolring erfolgt durch Umsetzung einer Verbindung der Formel IV mit Phosphoroxychlorid/Dimethylformamid (siehe Fig. 3b). Die Umsetzung wird in einem inerten Lösungsmittel, beispielsweise Benzol, Toluol oder Xylol, bei erhöhter Temperatur, zweckmäßigerweise am Siedepunkt des verwendeten Lösungsmittels, durchgeführt. Man erhält eine Verbindung der Formel IX, worin einer der Reste R¹, R² und R³ für CHO steht. Diese Formylgruppe kann dann in üblicher Weise, beispielsweise mit Lithiumaluminiumhydrid in einem inerten Lösungsmittel, beispielsweise Tetrahydrofuran, oder mit Natriumborhydrid in wäßrig alkalischer Lösung zu der entsprechenden Hydroxymethylverbindung XIX reduziert werden (Fig. 3b). Diese kann dann als Ausgangsmaterial für weitere Umsetzungen zur Einführung der gewünschten Gruppen dienen (Verfahren K, J; Fig. 3b),
   Weiter kann die Formylgruppe in einer unter üblichen Bedingungen durchgeführten Wittig-Reaktion in eine entsprechende Alkenylengruppe unter Bildung der Verbindung X überführt werden (siehe Verbindung X in Fig. 3b). Diese wiederum kann gewünschtenfalls in üblicher Weise zu der entsprechenden Alkylenverbindung (XXIII, Fig. 4) hydriert werden.
e) Umsetzung einer Verbindung der Formel IV mit einem Anhydrid der Formel: 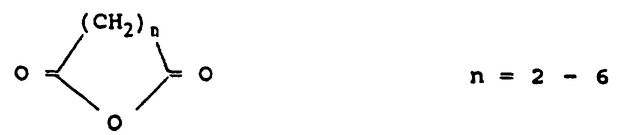 ergibt die entsprechenden Ketocarbonsäuren der Formel I, worin einer der Reste R¹, R² und R³ für CO(CH₂)ₙCO₂H steht. Die Ketocarbonylgruppe kann mit dem bereits erwähnten Reagens zu einer CH₂-Gruppe reduziert werden (siehe Fig. 3a, XI - XVI).
f) Eine Carboxylgruppe kann durch Umsetzung einer Verbindung der Formel IV mit n-Butyllithium in einem inerten Lösungsmittel bei tiefer Temperatur und anschließendes Durchleiten von CO₂-Gas durch die Lösung der gebildeten lithiumorganischen Verbindung eingeführt werden, man erhält Verbindungen der Formel XXII (siehe Verfahren L, Fig. 4).
g) Aus Carbonsäuren können durch Veresterung Ester und aus Estern durch Esterspaltung Carbonsäuren in üblicher Weise hergestellt werden.

Die Herstellung anderer erfindungsgemäßer Verbindungen erfolgt analog (Fig. 3a, 3b, 4), gegebenenfalls unter weiteren Umwandlungen, die dem Fachmann bekannt sind.

Die erfindungsgemäßen Verbindungen haben sich als potente Cyclooxygenase- und/oder Lipoxygenasehemmer erwiesen. Sie zeichnen sich durch eine starke analgetische Wirkung und durch eine gleichmäßige Hemmwirkung auf die Enzyme Cyclooxygenase (CO) und Lipoxygenase (LO) (IC₅₀LO/IC₅₀CO ∼ 1). Sie sind daher bei der Behandlung von Erkrankungen brauchbar, die mit einer Veränderung der Arachidonsäuremetabolisierung einhergehen. Insbesondere sind zu nennen Erkrankungen des rheumatischen Formenkreises und die Prävention von allergisch induzierten Erkrankungen. Die erfindungsgemäßen Verbindungen stellen somit wirksame Antiphlogistika, Analgetika, Antipyretika, Antiallergika dar und sind antibronchokonstriktorisch wirksam und daher zur Thromboseprophylaxe und zur Prophylaxe des anaphylaktischen und septischen Schocks sowie zur Behandlung dermatologischer Erkrankungen, wie Psoriasis, Urtikaria, akute und chronische Exantheme allergischer und nicht-allergischer Genese brauchbar. Außerdem sind sie zur Behandlung von Hypercholesterinämie brauchbar.

Die erfindungsgemäßen Verbindungen können entweder als einzelne therapeutische Wirkstoffe oder als Mischungen mit anderen therapeutischen Wirkstoffen verabreicht werden: Sie können als solche verabreicht werden, im allgemeinen werden sie jedoch in Form pharmazeutischer Mittel verabreicht, das heißt, als Mischungen der Wirkstoffe mit geeigneten pharmazeutischen Trägern oder Verdünnungsmitteln. Die Verbindungen oder Mittel können oral oder parenteral verabreicht werden, vorzugsweise werden sie jedoch in oralen Dosierungsformen gegeben.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z.B. Sirup Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglykol oder Siliciumdioxid), disintegrierende Mittel (z.B. Stärke) oder Netzmittel (z.B. Natriumlaurylsulfat). Orale flüssige Präparate können in Form wäßriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen.

Die erfindungsgemäßen Verbindungen oder Mittel können an ein Säugetier (Mensch und Tier) in Dosen von etwa 0,5 mg bis etwa 100 mg pro kg Körpergewicht pro Tag verabreicht werden. Sie können in einer Einzeldosis oder in mehreren Dosen verabreicht werden.

Die Wirksamkeit der erfindungsgemäßen Verbindungen läßt sich anhand der Hemmung der 5-Lipoxygenase oder der Cyclooxygenase bestimmen. Die Untersuchungen wurden nach Dannhardt et al.,J. Pharm. Pharmacol. 1992, 44:419-424 durchgeführt. Das Wirkungsspektrum der Verbindungen wurde außerdem anhand folgender Tests untersucht:
1) Phenylchinon-Writhing-Test an der Maus p.o., S. Irwin, Psychopharmacologia, 13:222-257, 1968;
2) Formalin-Analgesic-Test an der Maus p.o., B. Rubin et al., Endocrinol., 49:429-439, 1951;
3) Hemmung der Arachidonsäure-induzierten Plättchenaggregation, V. Bertele et al., Science 220:517-519 (1983);
4) Entzündungshemmung am Rattenpfotenödem, C.A. Winter et al., Proc. Exper. Biol. Med., 111:544-547 (1962);
5) Trachealrelaxierung am Meerschweinchen, F.P. Luduena et al., Arch. Int. Pharmacodyn., 111:392-400, 1957;
6) Cholesterinsenkende Wirkung an der Maus, C.E. Day et al., Atherosclerosis Drug Discovery, Edit. Charles E. Day, Plenum Publishing Corp., New York, 1976, 231-249.

Die Ergebnisse sind in der nachfolgenden Tabelle 1 angegeben:

**Tab. 1. :**

| **Pharmakologische Wirkung** | | | | | | | |
|---|---|---|---|---|---|---|---|
| **Verbindung**^{**1)**} | **Testmodelle** | | | | | | |
| | **IC50 LO/CO** | **1** | **2** | **3** | **4** | **5** | **6** |
| **1** | 4×10⁻⁷/2×10⁻⁷ | x | x | x | | x | |
| **8** | | x | | x | | x | |
| **9** | | x | x | x | | | |
| **10** | | | | x | | | x |
| **11** | | | | x | x | x | |
| **12** | | | | x | | | x |
| **6** | 4×10⁻⁸/×8.10⁻⁷ | | | | | | |
| **14** | | | | | | | x |

| | | | | | | | |
|---|---|---|---|---|---|---|---|
| ¹⁾ Nr. des Beispiels | | | | | | | |

Es wurde gefunden, daß die Verbindung des Beispiels 1 (R₂ = 5-Chlor-2-thienyl) gegenüber dem entsprechenden 4-Chlorphenylderivat in vivo 10mal stärker analgetisch wirksam ist. Im Formalin-Analgesie-Modell der Maus ergibt sich eine MED von 10 mg/kg im Vergleich zu 100 mg/kg.

Die nachfolgenden Beispiele erläutern die Erfindung. Alle Temperaturangaben sind unkorrigiert. Die IR-Spektren kristalliner Substanzen wurden als KBr-Preßlinge aufgenommen, die öliger Substanzen als Film. Die NMR-Spektren sind, sofern nicht anders vermerkt, 200 MHz-Spektren, aufgenommen in CDCl₃ mit Tetramethylsilan (TMS) als internem Standard. Die IR-Spektren sind in cm⁻¹ und die NMR-Spektren in δ(ppm) angegeben.

### Beispiele

### Allgemeine Herstellungsvorschrift für heteroarylsubstituierte [a]-bzw. [1,2]-anneliert Pyrrole (Pyrrolo[1,2-a]pyrrole = Pyrrolizine, Pyrrolo[1,2-a]pyridine = Indolizine, Pyrrolo[1,2-a]azepine)

Zu einer Lösung von 20 mmol ω-Bromacetylverbindung in 100 ml Methylenchlorid werden 20 mmol des entsprechenden cyclischen Imin-Derivates in 50 ml Methylenchlorid rasch zugetropft und bei Raumtemperatur 4 Stunden unter Feuchtigkeitsausschluß gerührt. Anschließend gibt man 30 ml 5%ige wäßrige NaHCO₃-Lösung zu und rührt intensiv weitere 4 Stunden. Nach Zugabe von 200 ml Wasser wird die organische Phase abgetrennt, über Na₂SO₄ getrocknet und unter vermindertem Druck verdampft. Der Rückstand wird mit Methanol zur Kristallisation gebracht und gegebenenfalls aus Methanol umkristallisiert.

### Allgemeine Herstellungsvorschrift für heteroarylsubstituierte [a]-bzw. [1,2]-annelierte Pyrrol-5-yl-oxoessigsäuren

Zu einer Lösung von 1,4 mmol Oxalsäureethylesterchlorid in 20 ml trockenem Methylenchlorid werden unter Rühren 1,3 mmol entsprechend substituierten annelierten Pyrrols, gelöst in 20 ml trockenem Methylenchlorid, unter Rühren zugetropft und 20 Minuten gerührt. Nach vorsichtigem Zusatz von 40 ml Wasser wird die organische Phase abgetrennt und über Na₂SO₄ getrocknet. Der nach Abzug des Lösungsmittels verbleibende Rückstand wird in 20 ml Diisopropylether suspendiert, abgesaugt und noch zwei mal mit je 5 ml Diisopropylether nachgewaschen.

### Allgemeine Herstellungsvorschrift für heteroarylsubstituierte [a]- bzw. [1,2]-annelierte Pyrrol-5-yl-essigsäuren

2 mmol des entsprechenden Oxoesterderivates werden mit 2 ml Diethylenglykol und 1,5 ml 80%igem Hydrazinderivat versetzt und 30 Minuten bei 60°C gerührt. Anschließend werden 2,1 g Kaliumhydroxid zugesetzt und das Reaktionsgemisch wird unter Rühren zwei Stunden auf 140°C erhitzt.

Das noch warme Gemisch wird auf 20 ml Eiswasser gegeben und mit verdünnter Phosphorsäure auf pH = 3 - 4 gebracht, wobei sich das Rohprodukt fest abscheidet. Man saugt ab, wäscht mehrfach mit Wasser nach, trocknet im Vacuum und wäscht anschließend mehrfach mit wenig Diisopropylether.

### Allgemeine Herstellungsvorschrift für N-sulfonylierte [a]-bzw. [1,2]-annelierte Heteroarylpyrrolcarbonsäureamide

### Mischung A:

2,6 mmol der betreffenden Pyrrolcarbonsäure werden mit 5 mmol Carbonyldiimidazol in 25 ml trockenem Tetrahydrofuran 1h bei Raumtemperatur gerührt.

### Mischung B:

3 mmol des entsprechend substituierten Sulfonamids werden unter Argon-Atmosphäre in 20 ml trockenem Tetrahydrofuran gelöst, mit 3,3 mmol Natriumhydrid (Mineralölsuspension) versetzt und 1h bei Raumtemperatur gerührt.

Mischung B wird unter Argon-Atmosphäre zu Mischung A gegeben und 40 h gerührt. Die Suspension wird auf 40 ml Eiswasser gegossen, mit verdünnter Phosphorsäure auf pH = 4 gebracht und mit Diethylether mehrfach extrahiert. Nach Trocknen der organischen Phase über Na₂SO₄ und Abziehen des Lösungsmittels, wird der verbleibende Rückstand aus Isopropanol umkristallisiert.

Die Synthese von 5,7-(hetero)arylsubstituierten Pyrrolizinen erfolgte analog EP-A-397 175.

Die entsprechenden Bromaldehyde wurden analog Riehl, J.J., C.R. Hebd. Seance, Acad. Sci. Ser. C, (1957), 245, S 1321 - 1322 hergestellt aus

| | |
|---|---|
| 2-Pyridylacetaldehyd | Leaver et al., J.Chem. Soc.[1963]6053. |
| 4-Pyridylacetaldehyd | Julia et al. J. Chem. Soc. Perkin Trans. 1 [1978] 1646-1650. |
| 3-Pyridylacetaldehyd | Hey, Williams, J. Chem. Soc. [1950]1678. |
| 2-Chinolinylacetaldehyd | analog Methode von Leaver et al. s.o. für 2-Pyridylacetaldehyd |
| 3-Indolylacetaldehyd | Plieninger, Weist; Chem. Ber. 89, 2783 [1956] Chem. Ber. 88, 1956 [1955] |
| 2-N-Methylpyrrolacetaldehyd | Hess, Merck, Uibig, Chem. Ber. 48, 1894 [1915] |
| 5-Chlor-2-thienylacetaldehyd | Mason, Nord; J. Org. Chem. 16, [1951] 1869-1871 |
| 2-Furanylacetaldehyd | Reichstein, Chem. Ber. 63, [1930]749-753 |

Die Einführung der A-Y-Reste in Position 6 erfolgt wieder analog EP-A-397 175.

Die erhaltenen Zwischenverbindungen und Endverbindungen sind in den nachfolgenden Tabellen 2 und 4 angegeben. Ihre physikalischen Daten folgen in den Tabellen 3 und 5.

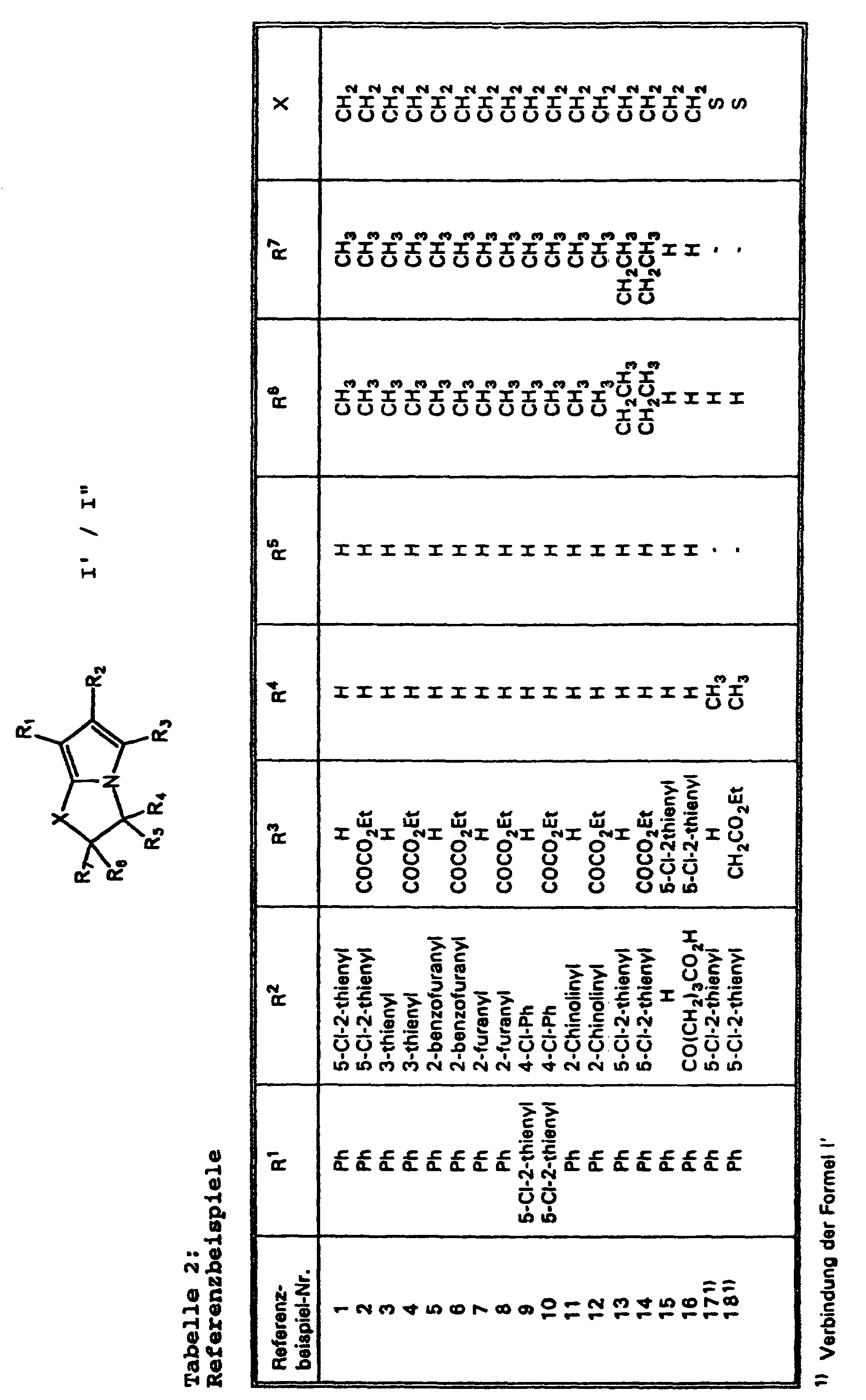

**Tabelle 3**

| **Verbindung des Referenzbeispiels-Nr.** | |
|---|---|
| **1** | Schmp.: Öl |
| | |
| | IR: 2950, 1656, 1596, 1444, 1414, 1382, 792, 759, 697 |
| | |
| | NMR: 7,29-7,17(m. 5H,arom); 6,71(s,1H,N-CH-); 6,70 (AB, J = 3,5, =CH-); 6,49 (AB,J=3,5,=CH-); 3,72(s,2H,-CH₂-N); 2,75(s,2H,-CH₂-); 1,27(s,6H,-CH₃) |
| **2** | Schmp.: 133,0°C |
| | |
| | IR: 2955, 1736, 1619, 1467, 1426, 1373, 1241, 1179, 1049, 701 |
| | |
| | NMR: 7.26-7,10(m, 5H, arom); 6,82 (AB. J=3,7, -CH=); 6,77 (AB, J=3,7, -CH=); 4,22(s,2H, -CH₂N-); 3.87(q, 2H, J = 7,0, ethyl); 2,82(s,2H, -CH₂-); 7,31(s,6H,-CH₂);1.19(t,3H,J=7,0,ethyl) |
| **3** | Schmp.: Öl |
| | |
| | IR: 2945, 1597, 1551, 1460, 1416, 1363, 1156, 757, 696 |
| | |
| | NMR: 7,27-7,17(m,5H, arom.); 6,97(s,1H),); 6,95(s,1H,); 6,72(s,1H,=CH-N-);3,73(s,2H,-CH₂N-); 2,78(s,2H. pyr.); 1,28(s,6H, -CH₃) |
| **4** | Schmp.: 136,4°C |
| | |
| | IR: 2950, 1732, 1609, 1450, 1420, 1249, 1132, 1062, 743 |
| | |
| | NMR: 7,26-6,88(m,8H, arom=ABX-thienyl);4,23(s,2H,-CH₂-N-); 3,75(q,2H,J =7,0,ethyl);2,85(s,2H,pyr);1,32(s,6H,-CH₃); 1,12(t,3H,J=7,0) |
| **5** | Schmp.: 145,2°C |
| | |
| | IR: 2945, 1601, 1462, 1276, 1249, 1162, 970, 790, 740, 698 |
| | |
| | NMR: 7,4-7,11(m,10H,arom.benzofuran); 6,31 (d,1H, = CH-N,J =0,76Hz); 3,77(s,2H,-CH₂-N-); 2,74(s,2H,-CH₂-); 1,28(s,6H,-CH₃₎ |
| **6** | Schmp.: 161°C |
| | |
| | IR: 2950, 1625, 1739, 1451, 1423, 1370, 1306, 1241, 1199, 1060 |
| | |
| | NMR: 7,53-7,17 (m,9H, arom, benzofuran); 6,58(d,1H, J =0,75Hz; 4,23(s,2H,-CH₂-N); 3,65(q,2H,J=7,2 Hz, ethyl); 2,83 (s,2H, -CH₂-) 1,33(s,6H,-CH₃); 0,98 (t,3H, J = 7,2 Hz, ethyl) |
| **7** | Schmp. 107,7 |
| | |
| | IR: 2940, 1600, 1478, 1368, 1176, 1008, 968, 753, 721, 698 |
| | |
| | NMR: 7,33-7,17(m,6H,arom,=CH-O); 6,88 (s,1H=CH-N-);6,30(dd,1H, J = 1,8 Hz); 6,01 (d. 1H, J = 3,2 Hz); 3,73 (s, 2H, -CH₂-N-), 2,74(s,2H,-CH₂- pyr.)); 1,29(s,6H,-CH₃) |
| **8** | Schmp. 129°C |
| | |
| | IR: 2975, 1719, 1616, 1443, 1262, 1181, 1062, 757, 692 |
| | |
| | NMR: 7,43-7,10(m,6H,arom=CH-O)); 6,41(dd,1H,J=1,9,);6,23(d,1H,J=3,2); 4,21(s,2H, -CH₂N-); 3,95(q,2H,J=7,1, ethyl); 2,84(s,2H,CH₂pyr); 1,32(s,6H,-CH₃); 1,15(t,3H,J=7,1, ethyl) |
| **9** | Schmp. 83°C |
| **10** | Schmp. 183°C |
| **11** | Schmp. Öl |
| | |
| | NMR: 8,30-8,05(m,3H,Chinolinyl); 7,95-7,7(m,2H); 7,65-7,25(m,6H,) 6,95(s,1H, Pyrrol); 3,88(s,2H,CH₂); 2,94(s;2H,CH₂); 1,31(s,6H,2CH₃) |
| **12** | Schmp. 153°C |
| | |
| | IR: 1740 |
| | |
| | NMR: 8,35-8,15 (m,3H, Chinolin). 8,0-7.3(m, 8H, Phenyl + 3HCh(inolin); 4,87(s;2H,CH₂),312(q,2H,OCH₂-CH₃); 2,95(s;2H,CH₂); 1,36(s 6H,2CH₃); 0,79(t,3H,CH₂-CH₃) |
| **13** | Schmp. 80,3°C |
| | |
| | IR: 2995, 1550, 1447, 1380, 1157, 1062, 986, 786, 758, 694 |
| | |
| | NMR: 7,30-7,15(m,5H,arom); 6,68(d,AB,1H, J=4,0, thienyl); 6,47 (d,AB,1H, thienyl) (6,67(s,1H,-N-CH=); 3,74(s,2H,-CH₂-N); 2,73(s,2H,-CH₂pyr); |
| **14** | Schmp.: 126,8 |
| | |
| | IR: C=O; 1750, 1629 |
| | |
| | NMR: 7,31-7,09 (m, 5H, arom); 6,81 +6,76(AB,2H, J=3,7,-CH=CH-) 4,23(s,2H,-CH₂-N-); 3,87(q,2H,J=7,2, ethylester); 2,79 (s,2H, CH₂) |
| **15** | Schmp.: 142°C |
| | |
| | NMR: 7,3 - 7,1 (m. 5H,Arn); 6,70 (H_{A},JS_{AB}=3,3), 6,49(H_{B}; J_{AB}=3,39) 6,66(s, 1H, Pyrrol H); 4,07 (t,2H; CH₂), 3,04 (t;2H; CH₂), 2.60 (q, 2H, CH₂) |
| **16** | Schmp. 126°C |
| | |
| | IR: 1660, 1706, (C=O) |
| | |
| | NMR: 8.7-8,5(2H, Ar); 8.5-8.35(3H;Ar); 6,82(H_{A}, J_{AB}=3,76Hz); 6,76 (H_{B}, J_{AB} = 3,75 Hz); 5,08 (t,2H, CH₂); 4,14(t,2H, CH₂); 3,67(quint.. 2H, CH₂); 3,53(t,2H, CH₂); 3,33 (t,2H, CH₂); 2,95 (quint.. 2H, CH₂). |
| **17** | NMR: 7,24-7,06 (m, 5H, Ar); 7,16 (s, 1H); 6,80, 6,62 (AB, J = 3,8 Hz, Thiophen-H); 6,31 (9, 1H, J = 0,8 Hz); 2,40 (d, 3H, J = 0,8 Hz) |
| **18** | NMR: 7,26-7,03 (m, 5H, Ar); 6,80, 6,62 (AB, J = 3,8 Hz, Thiphen-H); 6,30 (q, 1H, J = 0,8 Hz); 3,51 (s, 2H, CH₂); 2,62 q, 2H, 6,8 Hz); 2,38 (d, 3H, 0,8 Hz); 1,12 (t, 3H, J = 6,8 Hz) |

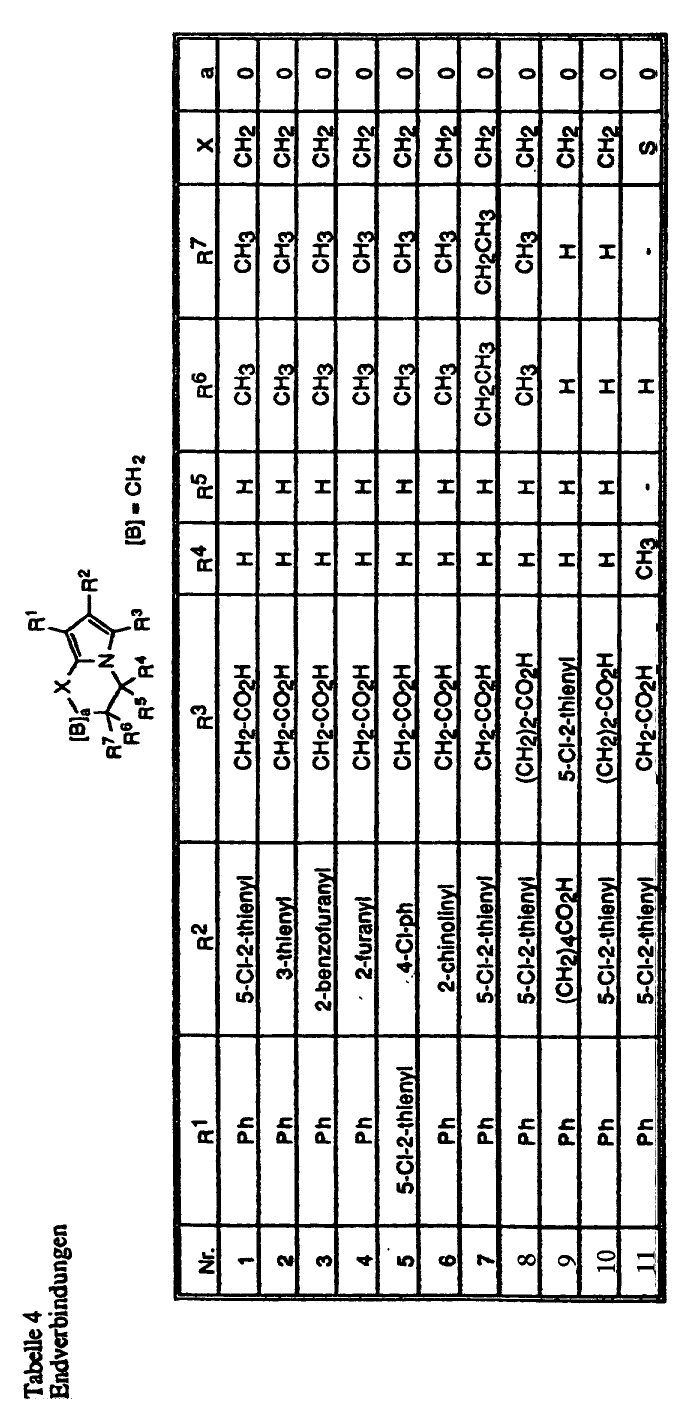

**Tabelle 5**

| **Verbindung des Beispiels Nr.** | |
|---|---|
| **1** | Schmp.: 164°C |
| | |
| | IR: 2920, 1706, 1599, 1441, 1417, 1250, 1222, 1056, 802, 694 |
| | |
| | NMR: 7,24-7.12(m, 5H, arom.); 6,80, 6,62 (AB, 3,8 Hz, Thiophen), 3,73 (s,2H, -CH₂N-); 3,67(s,2H, -CH₂-C=O); 2,83(s,2H,-CH₂-); 1,28(s,6H,-CH₃) |
| **2** | Schmp. : 157,2 °C |
| | |
| | IR:: 2950, 1700, 1598, 1447, 1412, 1307, 1270, 1224, 790, 685 |
| | |
| | NMR: 7.26-7,08 (m, 7H, arom. ABX-thienyl); 6,83 (dd.ABX, 1H, Thienyl); 3,76 (s, 2H, -CH₂-N-); 3,64 (s,2H,-CH₂-CO-); 2,85 (s,2H, -CH₂-); 1,30 (0,6H, -CH₃) |
| **3** | Schmp. :153,6 °C |
| | |
| | IR: 2950, 1707, 1600, 1451, 1416, 1251, 1217, 1163, 746, 696 |
| | |
| | NMR: 7,48-7.15(m,9H,arom. benzofuran); 6,43(d, 1H, J=0.6Hz CH=C-O); 3,89(s,2H,-CH₂-N-); 3,75(s,2H,-CH₂-C=O); 2,81(s,2H, -CH₂-pyr); 1,29(s,6H,-CH₃) |
| **4** | Schmp.: 164 °C |
| | |
| | IR: 3425, 2950, 1702, 1600, 1446, 1289, 1174, 1008, 758, 697 |
| | |
| | NMR: 7,37-7,10(m,6H,arom + =CH-O-); 6,37(dd, 1H, J= 1,9); 6,13 (d', 1H, J = 3,4) 3,75 (s,2H,); 3,73(s,2H,); 2,81 (s,2H, d); 1,28(5,6H, -CH₃) |
| **5** | Schmp.: 167°C |
| | |
| | IR: 1700 (C =O) |
| **6** | Schmp.: 173°C |
| | |
| | IR: 1703 |
| | |
| | NMR: 8,25-8,0 (m,3H, chinol.); 7.9-7.2 (m,8H,2Ar); 3,85 (s; 2H, CH₂); 3,78 (s. 2H, CH₂); 2,77 (s, 2H, CH₂), 1,30s,6H, 2CH₃); 12,0 (b, COOH) |
| **7** | Schmp.: 137,6°C |
| | |
| | IR: 2950, 1708, 1599, 1557, 1445, 1412, 1284, 1218, 801, 693, (C=O : 1720) |
| | |
| | NMR: 7,26-7,15 (m, 5H, arom.); 6,79+6,61(AB,2H,J=3,7, -CH= CH-); 3,76(s,2H,-CH₂-N-); 3,65 (s,2H,-CH₂C=O); 2,81(s,2H,-CH₂); 1,61(9 ,44,J=7,3,ethyl); 0,39(t,6H,J=7,3,ethyl) |
| **8** | Schmp. 199°C |
| | |
| | IR: 1704 (C = O) |
| | |
| | NMR: 7,23-7,11(m,5H,Ar); 6,80/6,61(AB,2H,J=3,8Hz, thien.); 3,71(s,2H, CH₂); 3,06(t,2H, CH₂, J=6,7 Hz), 2,79 (s,2H, CH₂), 2,36(t,2H,J=6,8Hz, CH₂); 1,24 (s, 6H, 2 CH₃) |
| **9** | Schmp.: 123°C |
| | |
| | IR: 1700 (C = O) |
| | |
| | NMR: 7,3-7,15(m, 5H; Ph); 6,70 (H_{A}. 1H, J_{AB}= 3.5 Hz), 6,50 (H_{B}, 1H, J_{AB}=3,5 Hz) 3,65 (t, 2H, CH₂), 2,88 (t, 2H, CH₂), 2,49/2,45 (2 quin. 4H, 2CH₂) 2,35 (t,2H, CH₂), 2,14 (t, 2H; CH₂); 1,77 (quin. 2H, CH₂ |
| **10** | IR: 1703 (C=O) |
| | |
| | NMR: 7,20-7,08 (m, 5H, arom.); 6,80, 6,62 (AB, 3,8 Hz, Thiophen); 2,31-2,64 (m, 4H, C-2 und CH₂-COOH); 2,70-3,10 (m, 4H, C-1 und Py-CH₂); 3,91 (t, 2H, J = 7 Hz, C -3) |
| **11** | NMR: 7,21-7,0 (m, 5H, Ar); 6,78, 6,61 (AB, J = 3,8 Hz, Thiophen-H); 6,30 (q, 1H, J = 0,8 Hz); 3,81 (s, 2H, CH₂); 2,41 (d,3H, J = 0,8 Hz) |

## Patentansprüche

1. Heterocyclische Verbindungen der Formel I: 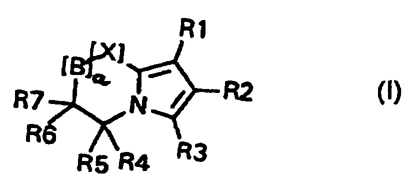 worin
einer der Reste R¹, R² und R³ für einen Thiophen-, Furan-, Benzofuran- oder Pyrimidinrest steht, der gegebenenfalls durch Halogen oder C₁-C₈-Alkyl substituiert ist,
der zweite der Reste R¹, R² und R³ für ein Wasserstoffatom oder einen Phenyl- oder Naphthylrest steht, der gegebenenfalls einen oder zwei Substituenten aufweist, die unter Halogen, Pseudohalogen, CF₃ und C₁-C₈-Alkyl ausgewählt sind, und
der dritte der Reste R¹, R² und R³ für H, CHO, CO₂H, COSC₁-C₈-Alkyl, COCO₂H, oder A - Y steht,
A für C₁-C₈-Alkylen oder C₂-C₈-Alkenylen steht,
Y für CO₂H, SO₃H, OPO(OH)₂, OP(OH)₂, eine Tetrazolylgruppe, CHO oder OH steht,
R⁴, R⁵, R⁶ und R⁷, die gleich oder verschieden sein können, für H oder C₁-C₈-Alkyl stehen oder zwei dieser Reste für eine chemische Bindung zwischen den beiden Ringatomen, an die sie gebunden sind, stehen und die beiden anderen die angegebenen Bedeutungen besitzen,
X für CH₂ oder S steht,
B für CH₂ steht, und
a für 0, 1 oder 2 steht, und
die optischen Isomere, Salze und C₁-C₈-Alkyl-, Pivaloyloxymethyl-, Acetoxymethyl-, Phthalidyl-, Indanyl- oder Methoxymethylester davon.

2. Verbindungen nach Anspruch 1, wobei einer der Reste R¹, R² und R³ für den erwähnten heterocyclischen Rest steht und der zweite für Phenyl oder durch ein bis drei Halogenatome oder CF₃ substituiertes Phenyl steht.

3. Verbindungen nach einem der Ansprüche 1 oder 2, wobei der dritte der Reste R¹, R² und R³ für A - Y steht

4. Verbindung nach Anspruch 1, nämlich 6-(Benzo[b]furan-2-yl)-2,2-dimethyl-7-phenyl-2,3-dihydro-1H-pyrrolizin-5-yl-essigsäure.

5. Pharmazeutisches Mittel, enthaltend wenigstens eine Verbindung der Formel I, worin der erste und zweite der Reste R¹, R² und R³ wie in Anspruch 1 definiert sind und der dritte der Reste R¹, R² und R³ für A - Y steht, wobei Y für CO₂H, SO₃H, OPO(OH)₂, OP(OH)₂ oder eine Tetrazolylgruppe steht, und A, R⁴, R⁵, R⁶, R⁷, X, B und a die in Anspruch 1 angegebenen Bedeutungen besitzen, oder ein optisches Isomer, ein pharmazeutisch verträgliches Salz oder die optischen Isomere, Salze und C₁-C₈-Alkyl-, Pivaloyloxymethyl-, Acetoxymethyl-, Phthalidyl-, Indanyl- oder Methoxymethylester davon, gegebenenfalls in Kombination mit pharmazeutisch verträglichen Träger- und/oder Zusatzstoffen.

6. Verwendung wenigstens einer Verbindung wie in Anspruch 5 definiert zur Herstellung eines pharmazeutischen Mittels zur Prävention von allergisch induzierten Erkrankungen oder zur Behandlung von Erkrankungen des rheumatischen Formenkreises.

7. Verfahren zur Herstellung der Verbindungen nach einem der Ansprüche 1 bis 4, **dadurch gekennzeichnet, daß** man
eine Verbindung der allgemeinen Formel II: 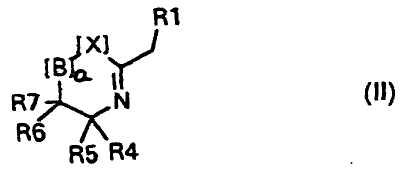 mit einer Verbindung der allgemeinen Formel III: 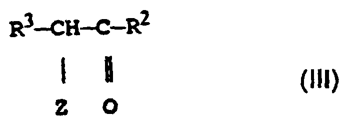 wobei in den obigen Formeln zwei der Reste R¹, R² und R³ die in Anspruch 1 angegebenen Bedeutungen besitzen und der dritte für ein Wasserstoffatom steht und Z für Cl oder Br steht, zu einer Verbindung der allgemeinen Formel la: 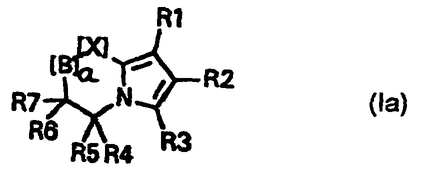 worin R¹ bis R⁷, B, a und X die oben angegebenen Bedeutungen besitzen, umsetzt.

8. Verfahren nach Anspruch 7, **dadurch gekennzeichnet, daß** man zur Herstellung der Verbindungen der Formel 1, worin der dritte der Reste R¹, R² und R³ für CH₂COOH, CH₂COOC₁-C₈-Alkyl oder COCO₂H steht, eine Verbindung der in Anspruch 7 definierten Formel la
a) mit Oxalylchlorid zu einer Verbindung der Formel I umsetzt, in der einer der Reste R¹, R² und R³ für COCO₂H steht, und gewünschtenfalls diese Verbindung mit einem Reagens behandelt, das zur Reduktion der Ketogruppe der Ketocarbonsäure zu einer CH₂-Gruppe geeignet ist, so daß man eine Verbindung der Formel I erhält, in der einer der Reste R¹, R² und R³ für CH₂CO₂H steht,
b) mit einem Diazoessigsäurealkylester zu einer Verbindung der Formel I umsetzt, in der einer der Reste R¹, R² und R³ für CH₂COOC₁-C₈-Alkyl steht, und gewünschtenfalls die erhaltene Verbindung einer Esterspaltung unterwirft, um eine Verbindung der Formel I zu erhalten, in der einer der Reste R¹, R² und R³ für CH₂CO₂H steht, oder
c) mit Chloral zu einer Verbindung der Formel I umsetzt, in der einer der Reste R¹, R² und R³ für -CH(OH)CCl₃ steht, und die erhaltene Verbindung in ein aktiviertes Derivat überführt und dieses mit Dithionit zu einer Verbindung der Formel I reduziert, in der einer der Reste R¹, R² und R³ für CH₂COOH steht.

9. Verfahren zur Herstellung der Verbindungen der Formel Ib 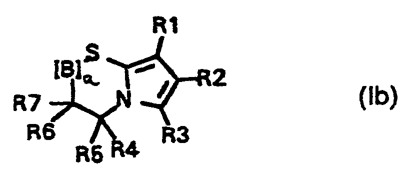 worin X für S steht, **dadurch gekennzeichnet, daß** man eine Verbindung der Formel VI: 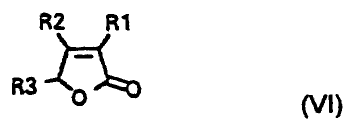 worin R¹, R² und R³ die in Anspruch 1 angegebenen Bedeutungen besitzen, mit einer Verbindung der Formel 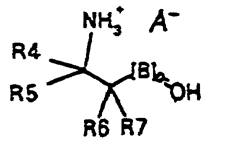 worin A für ein Anion steht, B, a und R⁴ bis R⁷ die in Anspruch 1 genannten Bedeutungen besitzen, zu einer Verbindung der Formel VII 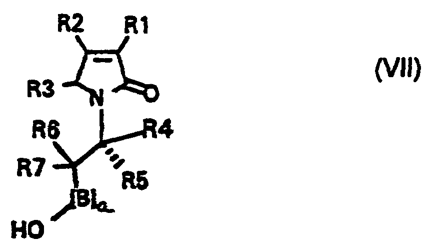 umsetzt und die Verbindung der Formel VII in Anwesenheit von Phosphorpentasulfid zu einer Verbindung der Formel Ib 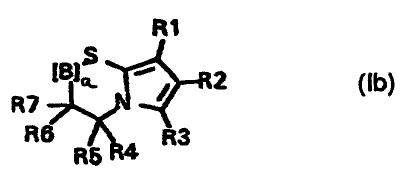 cyclisiert.

10. Verfahren zur Herstellung der Verbindungen der Formel l' 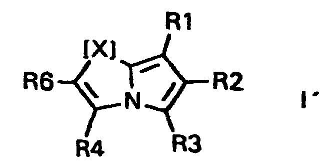 worin R¹ bis R⁶ die in Anspruch 1 angegebenen Bedeutungen besitzen und X für S steht,
**dadurch gekennzeichnet, daß** man eine Verbindung der in Anspruch 9 definierten Formel VI mit einer Verbindung der Formel 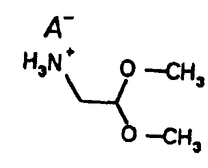 worin A⁻ für ein Anion steht, zu einer Verbindung der Formel VIII 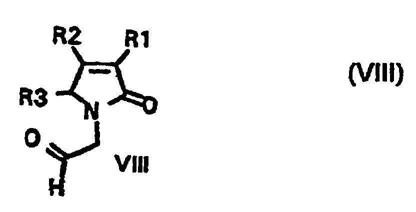 umsetzt und
eine Verbindung der Formel VIII mit Phosphorpentasulfid zu einer Verbindung der Formel l' cyclisiert.

## Claims

1. Heterocyclic compounds of formula I: 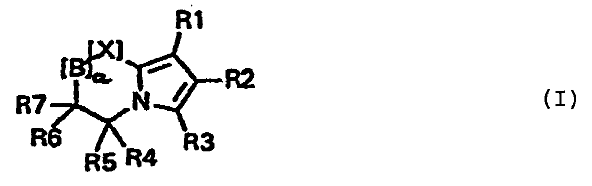 wherein
one of the groups R¹, R² and R³ denotes a thiophenyl, furanyl, benzofuranyl or pyrimidinyl group, optionally substituted by halogen or C₁-C₈-alkyl,
the second of the groups R¹, R² and R³ denotes a hydrogen atom or a phenyl or naphthyl group, optionally having one or two substituents selected from halogen, pseudohalogen, CF₃ and C₁-C₈-alkyl, and
the third of the groups R¹, R² and R³ denotes hydrogen, CHO, CO₂H, COSC₁-C₈-alkyl, COCO₂H, or A-Y,
A denotes C₁-C₈ alkylene or C₂-C₈ alkenylene
Y denotes CO₂H, SO₃H, OPO(OH)₂, OP(OH)₂, a tetrazolyl group, CHO or OH,
R⁴, R⁵, R⁶ and R⁷, which may be the same or different, denote H or C₁-C₈ alkyl or two of these groups denote a chemical bond between the two ring atoms to which they are bonded, the two other groups having the meanings as given,
X denotes CH₂ or S
B denotes CH₂ and
a denotes 0, 1 or 2 and
the optical isomers, salts and C₁-C₈ alkyl, pivaloyloxymethyl, acetoxymethyl, phthalidyl, indanyl or methoxymethylesters thereof.

2. Compounds according to claim 1, wherein one of the groups R¹, R² and R³ denotes the mentioned heterocyclic group and the second denotes phenyl or phenyl substituted by 1 to 3 halogen atoms or CF₃.

3. Compounds according to one of claims 1 or 2, wherein the third of the groups R¹, R² and R³ denotes AY.

4. A compound according to claim 1, namely 6-(benzo[b]furan-2-yl)-2,2-dimethyl-7-phenyl-2,3-dihydro-1H-pyrrolizin-5-yl-acetic acid.

5. A pharmaceutical agent containing at least one compound of formula 1, wherein the first and second of the groups R¹, R² and R³ are as defined in claim 1 and the third of the groups R¹, R² and R³ denotes A-Y, wherein Y denotes CO₂H, SO₃H, OPO(OH)₂, OP(OH)₂ or a tetrazolyl group and A, R⁴, R⁵, R⁶, R⁷, X, B and a have the meanings given in claim 1, or an optical isomer, a pharmaceutically-compatible salt or the optical isomers, salts and C₁-C₈ alkyl, pivaloyloxymethyl, acetoxymethyl, phthalidyl, indanyl or methoxymethylesters thereof, optionally in combination with pharmaceutically-compatible carriers and/or additives.

6. Use of at least one compound as defined in claim 5 for the production of a pharmaceutical agent for the prevention of allergically-induced illnesses or for the treatment of rheumatic illnesses.

7. Process for the production of compounds according to claims 1 to 4, **characterised in that** a compound of general formula II 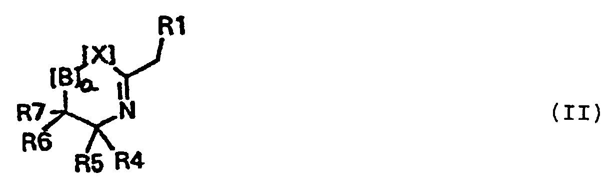 is reacted with a compound of general formula III 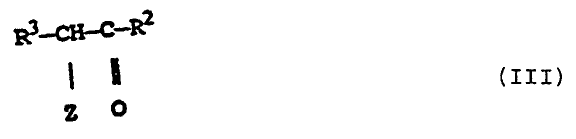 wherein in the above formulae, two of the groups R¹, R² and R³ have the meanings as given in claim 1 and the third denotes a hydrogen atom and Z denotes Cl or Br, to give a compound of formula Ia 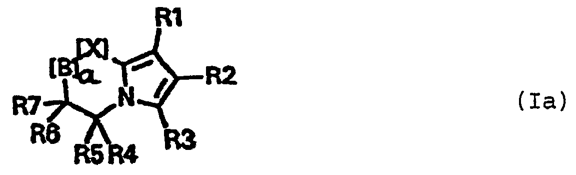 wherein R¹ to R⁷, B, a and X are as defined above.

8. Process of claim 7 **characterised in that** to prepare a compound of formula I, wherein the third of the groups R¹, R² and R³ denotes CH₂COOH, CH₂COOC₁-C₈ alkyl or COCO₂H, a compound of formula 1a, as defined in claim 7 is reacted:
(a) with oxalylchloride to give a compound of formula I in which one of the groups R¹, R² and R³ denotes COCO₂H, and this compound is optionally treated with a reagent which is suitable for reducing the keto group of the keto carboxylic acid to a CH₂ group so that one obtains a compound of formula 1 wherein one of the groups R¹, R² and R³ denotes CH₂CO₂H,
(b) with a diazoacetic acid alkylester to give a compound of formula I wherein one of the groups R¹, R² and R³ denotes CH₂COOC₁-C₈-alkyl, and optionally subjecting the obtained compound to ester cleavage in order to obtain a compound of formula I wherein one of the groups R¹, R² and R³ denotes CH₂CO₂H, or
(c) with chloral to give a compound of formula 1, wherein one of the groups R¹, R² and R³ denotes -CH(OH)CCl₃ and changing the obtained compound into an activated derivative and reducing this with dithionite into a compound of formula 1 wherein one of the groups R¹, R² and R³ denotes CH₂COOH.

9. A process for the production of a compound of formula Ib 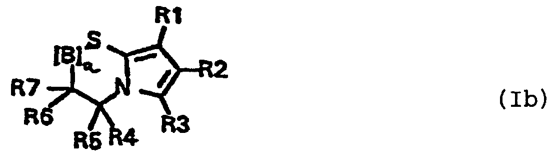 wherein X denotes S, **characterised in that** a compound of formula (VI) 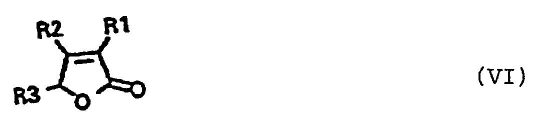 wherein R¹, R² and R³ have meanings as defined in claim 1, is reacted with a compound of formula 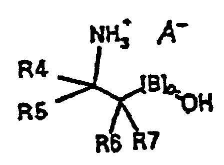 wherein A denotes an anion and B, a and R⁴ to R⁷ have the meanings given in claim 1, to give a compound of formula VII 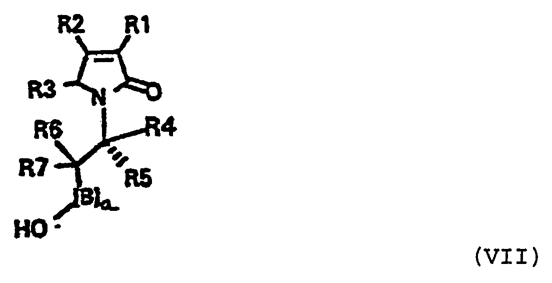 and the compound of formula VII is cyclised in the presence of phosphorus pentasulphide to give a compound of formula Ib

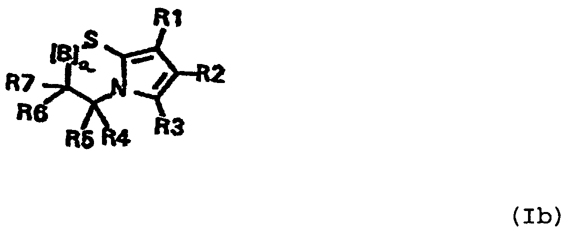

10. A process for the production of a compound of formula I' 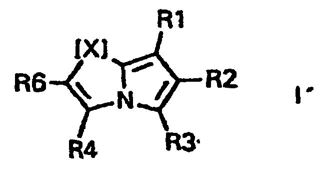 wherein R¹ to R⁶ have the meanings as given in claim 1 and X denotes S,
**characterised in that** a compound as defined in claim 9 of formula VI is reacted with a compound of formula 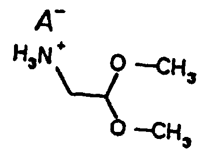 wherein A⁻ denotes an anion, to give a compound of formula VIII 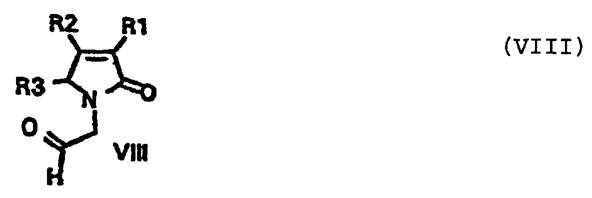 and cyclising a compound of formula VIII with phosphorus pentasulphide to give a compound of formula I'.

## Revendications

1. Composés hétérocycliques de formule I : 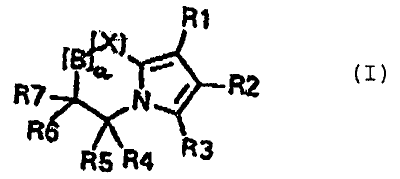 dans laquelle l'un des restes R¹, R² et R³ représente un reste thiophène, furanne, benzofuranne ou pyrimidine, qui est éventuellement substitué par un atome d'halogène ou un groupe alkyle en C₁ à C₈,
un deuxième des restes R¹, R² et R³ représente un atome d'hydrogène ou un groupe phényle ou naphtyle qui porte éventuellement un ou deux substituants qui sont choisis parmi les atomes d'halogène, les pseudohalogènes, CF₃ et les groupes alkyle en C₁ à C₈,
et le troisième des restes R¹, R² et R³ représente H, CHO, CO₂H, COS-alkyl(C₁ à C₈), COCO₂H ou A-Y où A représente un groupe alkylène en C₁ à C₈ ou un groupe alcénylène en C₂ à C₈ et Y représente CO₂H, SO₃H, OPO(OH)₂, OP(OH)₂, CHO, OH ou un groupe tétrazolyle,
R⁴, R⁵, R⁶ et R⁷ qui peuvent être identiques ou différents, représentent chacun H ou un groupe alkyle en C₁ à C₈, ou bien deux de ces restes représentent une liaison chimique entre les deux atomes du cycle auxquels ils sont liés et les deux autres restes ont les significations indiquées,
X représente CH₂ ou S,
B représente CH₂, et
a est égal à 0, 1 ou 2,
et les isomères optiques, sels et esters d'alkyle (C₁ à C₈), de pivaloyloxyméthyle, d'acétoxyméthyle, de phtalidyle, d'indanyle ou de méthoxyméthyle de ces composés.

2. Composés selon la revendication 1, pour lesquels l'un des restes R¹, R² et R³ représente le reste hétérocyclique mentionné et un deuxième représente un groupe phényle ou un groupe phényle substitué par 1 à 3 atomes d'halogène ou groupes CF₃.

3. Composés selon la revendication 1 ou 2, pour lesquels le troisième des restes R¹, R² et R³ représente A-Y.

4. Composé selon la revendication 1, qui est l'acide 6-(benzo[b]furan-2-yl)-2,2-diméthyl-7-phényl-2,3-dihydro-1H-pyrrolizin-5-yl-acétique.

5. Agent pharmaceutique qui contient au moins un composé de formule I pour lequel le premier et le deuxième des restes R¹, R² et R³ sont définis comme dans la revendication 1 et le troisième des restes R¹, R² et R³ représente A-Y où Y représente CO₂H, SO₃H, OPO(OH)₂, OP(OH)₂ ou un groupe tétrazolyle, et A, R⁴, R⁵, R⁶, R⁷, X, B et a ont les significations indiquées dans la revendication 1, ou un isomère optique, un sel pharmaceutiquement acceptable ou les isomères optiques, sels et esters d'alkyle (C₁ à C₈), de pivaloyloxyméthyle, d'acétoxyméthyle, de phtalidyle, d'indanyle ou de méthoxyméthyle de ce composé, éventuellement en combinaison avec des supports et/ou additifs pharmaceutiquement acceptables.

6. Utilisation d'au moins un composé tel que défini dans la revendication 5 pour la préparation d'un agent pharmaceutique destiné à la prévention des maladies d'origine allergique ou au traitement des maladies du domaine rhumatismal.

7. Procédé de préparation des composés selon l'une quelconque des revendications 1 à 4, **caractérisé en ce que** l'on fait réagir un composé de formule générale II : 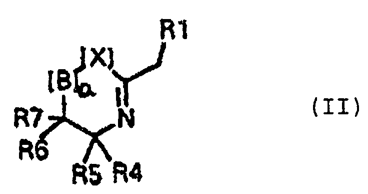 avec un composé de formule générale III : 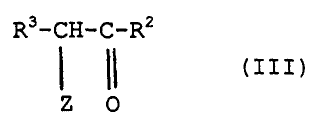 formules dans lesquelles deux des restes R¹, R² et R³ ont les significations indiquées dans la revendication 1 et le troisième représente un atome d'hydrogène et Z représente Cl ou Br, pour obtenir un composé de formule générale Ia : 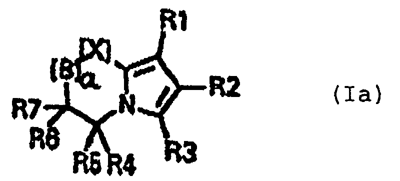 dans laquelle R¹ à R⁷, B, a et X ont les significations indiquées ci-dessus.

8. Procédé selon la revendication 7, **caractérisé en ce que**, pour préparer les composés de formule I pour lesquels le troisième des restes R¹, R² et R³ représente CH₂COOH, CH₂COO alkyl(C₁ à C₈) ou COCO₂H,
a) on fait réagir un composé de formule Ia définie dans la revendication 7 avec du chlorure d'oxalyle pour obtenir un composé de formule I dans lequel l'un des restes R¹, R² et R³ représente COCO₂H, et on traite éventuellement ce composé avec un réactif qui convient pour réduire le groupe cétonique de l'acide cétocarboxylique en un groupe CH₂, de sorte que l'on obtient un composé de formule I dans lequel l'un des restes R¹, R² et R³ représente CH₂CO₂H,
b) on fait réagir un composé de formule Ia définie dans la revendication 7 avec un ester d'alkyle de l'acide diazoacétique pour obtenir un composé de formule I dans lequel l'un des restes R¹, R² et R³ représente CH₂COO alkyl(C₁ à C₈), et on soumet éventuellement le composé obtenu à une coupure de l'ester afin d'obtenir un composé de formule I dans lequel l'un des restes R¹, R² et R³ représente CH₂CO₂H, ou bien
c) on fait réagir un composé de formule Ia définie dans la revendication 7 avec du chloral pour obtenir un composé de formule I dans lequel l'un des restes R¹, R² et R³ représente -CH(OH)CCl₃, on transforme le composé obtenu en un dérivé activé et on réduit ce dernier avec du dithionite pour obtenir un composé de formule I dans lequel l'un des restes R¹, R² et R³ représente CH₂COOH.

9. Procédé de préparation des composés de formule Ib 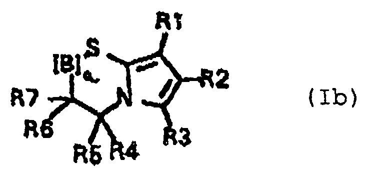 **caractérisé en ce que** l'on fait réagir un composé de formule VI : 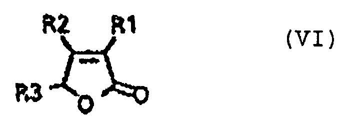 dans laquelle R¹, R² et R³ ont les significations indiquées dans la revendication 1, avec un composé de formule 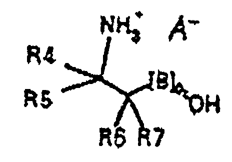 dans laquelle A représente un anion et B, a et R⁴ à R⁷ ont les significations indiquées dans la revendication 1, pour obtenir un composé de formule VII 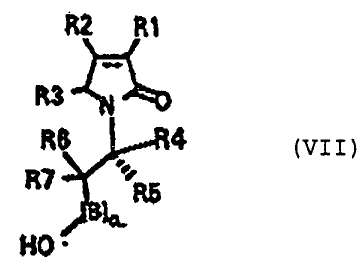 et on cyclise le composé de formule VII en présence de pentasulfure de phosphore pour obtenir un composé de formule Ib

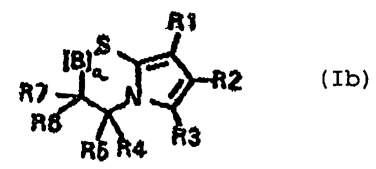

10. Procédé de préparation des composés de formule I' 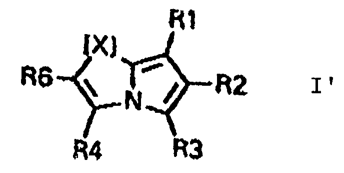 dans laquelle R¹ à R⁶ ont les significations indiquées dans la revendication 1 et X représente S, **caractérisé en ce que** l'on fait réagir un composé de formule VI définie dans la revendication 9 avec un composé de formule 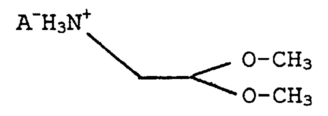 dans laquelle A⁻ représente un anion, pour obtenir un composé de formule VIII 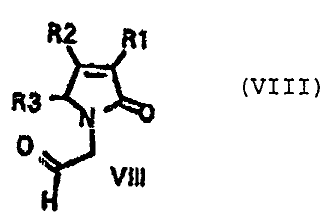 et on cyclise le composé de formule VIII avec du pentasulfure de phosphore pour obtenir un composé de formule I'.
